# EUROPEAN PATENT APPLICATION

(11) **EP 4 781 960 A1**
(43) Date of publication of application: **29.07.2026**
(21) Application number: 26153442.4
(22) Date of filing: 22.01.2026
(51) Int. Cl.: A61F 13/15, B65H 57/16

(54) **SYSTEM AND METHOD FOR FEEDING ELASTIC STRANDS**

(30) Priority: 22.01.2025 US 202519033903
(71) Applicant: First Quality Baby Products, LLC, Great Neck, NY 11021 (US)
(72) Inventor: Cappellini, Pierluigi, Bellefonte, 16823 (US)
(74) Representative: Modiano, Gabriella Diana

(57) **Abstract**

An elastic strand conveyance system that includes stack plates disposed on linear actuators. The stack plates include a base plate that supports entry and exit yarn guides. Wheels and pitch plates are disposed between the entry and exit yarn guides. Elastic strands are conveyed through the entry and exit yarn guides and over the wheels at a pitch determined by the pitch plates. The stack plates are capable of being disengaged from the linear actuators to allow for manual movement of the stack plates along rails of the linear actuators so that elastic strands may be replaced in the case of breakage.

## Description

### TECHNICAL FIELD

The present invention is directed to systems and methods for feeding elastic strands in a manufacturing environment, and in particular to systems and methods for feeding elastic strands to form elastic panels of absorbent articles.

### BACKGROUND

Diaper or protective underwear elastic panels typically contain many elastic strands. Some absorbent products typically include two elastic panels, one in front and the other in the back. The elastic panels are attached to a chassis and then attached to each other at side portions. Each panel may include many elastic strands, for example some front panels have 30 elastic strands and some back panels have 80 elastic strands. Elastic strands are known as Spandex or Elastane and are sold for example under the LYCRA brand sold by the Lycra company. In order to make the elastic panels, elastic strands are fed off stands, each holding typically two or more elastic cores. In order to supply a sufficient number of strands, elastic core containing stands are typically stacked for example six or more stands high. While it may be possible to have five or less stands stacked, it would require more floor space, which typically is at a premium in a manufacturing facility. The height of a stack of stands may reach seven feet or more. The lower stands typically direct elastic strands up to a common point near the highest stand before turning the strands 90 degrees such that they travel horizontally side by side towards a lamination station where the strands will be attached between nonwovens to form elastic panels. There are multiple stacks of elastic strand stands side by side feeding strands to the panels. With so many elastic strands feeding into the lamination station, it may be difficult to find and restring a broken elastic strand. In the path to the lamination station, the strands typically are re-directed (for example up, then down, then lateral, then up and down again) many times over rollers.

At the lamination station, the elastic strands are turned again 90 degrees to align with the nonwovens. The lamination station may make front and back elastic panels, side by side. Some elastic strands are fed to the front panel and others are fed to the back panel. If strands break, machine operators need to be able to access the strands to restring the strands and restart the machine. This creates potentially hazardous situations where the operator can fall. Where the elastic strand turns to be directed into the lamination station, it can be difficult to see rollers or wheels the strands are guided over to properly place the strands. There is a need for an improved elastic strand feeding system.

### SUMMARY OF THE INVENTION

An object of the present invention is to provide a system that delivers elastic strand feeding at a height where operators minimize the need for a ladder to reach and replace the strands. The system is arranged such that elastic strands run horizontally at approximately 5 feet from the floor. The height can vary based on the typical height of the machine operators, the feeding source of strands or the area in the plant where the strands are running. The elastic strands can be fed from a drive side, an operator side or both.

In accordance with exemplary embodiments of the present invention, a stack plate for directing elastic strands to a lamination station of an absorbent article manufacturing line comprises: a base block; one or more entry yarn guides disposed on the base block and each configured to receive a corresponding elastic strand of one or more elastic strands; one or more wheels disposed on the base block and each corresponding to one of one or more entry yarn guides and over which the corresponding elastic strand is guided as the one or more wheels rotate; one or more exit yarn guides disposed on the base block and each corresponding to one of the one or more entry yarn guides and to one of the one or more wheels, each of the one or more exit yarn guides configured to guide the corresponding elastic strand away from the stack plate; and one or more pitch plates disposed on the base block and each corresponding to one of the one more entry yarn guides, one of the one or more wheels and one of the one or more exit yarn guides, each of the one or more pitch plates extending between the corresponding one of the one or more wheels and the corresponding one of the one or more exit yarn guides, each of the one or more pitch plates having lengths that differ from one another so as to determine a distance between the one or more elastic strands.

In exemplary embodiments of the present invention, the stack plate further comprises a rod extending perpendicular to the one or more pitch plates, the rod configured to hold the one or more pitch plates in position on the base block.

In exemplary embodiments of the present invention, each of the one or more stack plates comprise openings, and the rod extends through the openings.

In exemplary embodiments of the present invention, the rod comprises a removeable end portion to allow for placement of the one or more pitch plates on the rod.

In exemplary embodiments of the present invention, each of the one or more wheels are disposed on the corresponding one of the one or more pitch plates.

In exemplary embodiments of the present invention, the one or more entry yarn guides, the one more wheels and the one or more exit yarn guides are positioned so that the one or more elastic strands are turned 90 degrees between the one or more entry yarn guides and the one or more exit yarn guides.

In exemplary embodiments of the present invention, the one or more wheels comprise an inner core member that forms a flat surface on which the one or more elastic stands are conveyed as the one or more wheels rotate.

In exemplary embodiments of the present invention, the stack plate is configured for placement on a linear actuator. Sets of stack plates are configured on the linear actuator.

In accordance with exemplary embodiments of the present invention, an elastic strand conveyance system comprises: (A) one or more alignment and lowering systems for directing elastic strands to one or more stack plates of a lamination station of an absorbent article manufacturing line, each of the one or more stack plates comprising: (i) a base block; (ii) one or more entry yarn guides disposed on the base block and each configured to receive a corresponding elastic strand of one or more elastic strands; (iii) one or more wheels disposed on the base block and each corresponding to one of the one or more entry yarn guides and over which the corresponding elastic strand is guided as the one or more wheels rotate; (iv) one or more exit yarn guides disposed on the base block and each corresponding to one of the one or more entry yarn guides and to one of the one or more wheels, each of the one or more exit yarn guides configured to guide the corresponding elastic strand away from the stack plate; and (v) one or more pitch plates disposed on the base block and each corresponding to one of the one or more entry yarn guides, one of the one or more wheels and one of the one or more exit yarn guides, each of the one or more pitch plates extending between the corresponding one of the one or more wheels and the corresponding one of the one or more exit yarn guides, each of the one or more pitch plates having lengths that differ from one another so as to determine a distance between the one or more elastic strands; (B) one or more linear actuators to which the one or more stack plates are operatively attached to allow for linear movement of the one or more stack plates.

In exemplary embodiments of the present invention, each of the one or more linear actuators comprises a rail on which a corresponding one of the one or more stack plates is conveyed in a linear motion by the linear actuator.

In exemplary embodiments of the present invention, the one or more stack plate are linearly disengageable from the rails of the one or more linear actuators to allow for manual movement of the one or more stack plates.

In exemplary embodiments of the present invention, the one or more stack plates comprise a plurality of stack plates.

### BRIEF DESCRIPTION OF THE DRAWINGS

FIG. 1 is a perspective view of a stack plate according to an exemplary embodiment of the present invention;
FIG. 2 is an exploded view of a stack plate according to an exemplary embodiment of the present invention;
FIG 3A is a cross sectional view of a conventional wheel for conveying an elastic strand;
FIG. 3B is a cross sectional view of a wheel for conveying an elastic strand according to an exemplary embodiment of the present invention;
FIG. 4 is a perspective view of an elastic strand conveyance system according to an exemplary embodiment of the present invention;
FIG. 5A is a perspective view of a linear actuator according to an exemplary embodiment of the present invention;
FIG. 5B is a perspective view of a linear actuator according to an exemplary embodiment of the present invention;
FIG. 6 is a perspective view of an elastic strand conveyance system according to an exemplary embodiment of the present invention; and
FIG. 7 is a perspective view of an elastic strand conveyance system according to an exemplary embodiment of the present invention.
FIGs. 8A and 8B are a side views of a alignment and lowering system according to an exemplary embodiment of the present invention.
FIG. 9A is a side view of an upper horizontal support bar according to an exemplary embodiment of the present invention.
FIG. 9B is a side view of a lower horizontal support bar according to an exemplary embodiment of the present invention.

### DETAILED DESCRIPTION

There are several aspects of the present invention relating to preventing strands from breaking and retrieving broken strands. Use of ceramic yarn guides keep elastic strands aligned with the center of rollers or wheels. This prevents elastic strands from contacting sides of the rollers or wheels and avoids twisting. Commercially available v-shaped rollers or wheels lead to twisting. Rollers or wheels associated with exemplary embodiments of the present invention have flat inner surfaces to help avoid twisting. The flat rollers or wheels also prevent elastic strands from jumping off rollers. Brushes are placed along a path the elastic strands follow to catch snapback of broken elastic strands.

Each strand is pulled off the core by a motor and passed around a sensor to control strain on the elastic strand. In accordance with exemplary embodiments of the present invention, the strands are fed up to an alignment and lowering system, which spaces the elastic strands and brings them back down to the height in which they run to stack plates on a linear actuator at the lamination station. The alignment and lowering system includes a vertical support bar with upper and lower horizontal support bars. The alignment and lowering system is attached to an upper portion of the stack of elastic core stands at a slight angle. Attached to the horizontal upper support bar is a rod with at least one wheel. On a lower portion of the upper support bar is a yarn guide mount with at least on yarn guide. Elastic strands pass through the yarn guides and over the wheels that turn elastic strands back down at least one wheel attached to the lower horizontal support bar which turns the elastic strand to run horizontally at the height the strands will travel horizontally. The elastic strand then passes through a yarn guide on a yarn guide mount (attached to the horizontal lower support bar) and under brushes (also attached to the lower horizontal support bar) that catch any broken elastic strands and prevent further snap back of the strands. The turning wheels are the same pitch (space between wheels). The height of the vertical support bar is dependent on the distance the elastic strand needs to be lowered to establish the desired horizontal elastic strand run height.

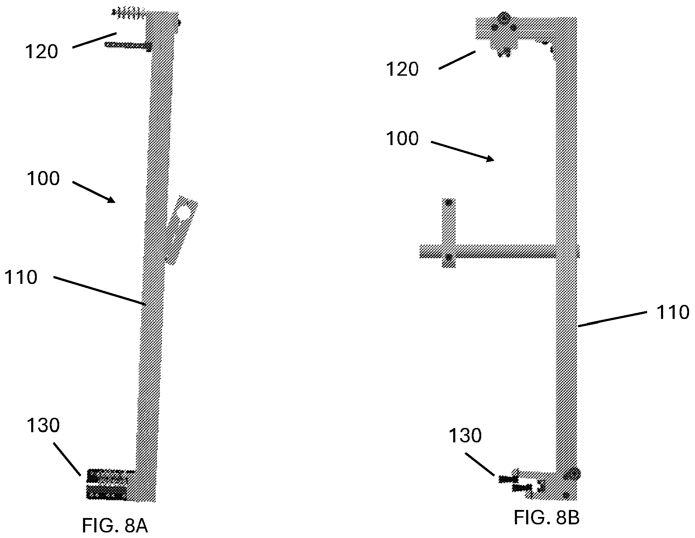

FIGS. 8A and 8B show a strand guide bar, generally designated by reference number 100, that is a component of an alignment and lowering system according to an exemplary embodiment of the present invention. The strand guide bar 100 includes a vertical support bar 110, a first strand guide 120 disposed at a top end portion of the vertical bar 110 and a second strand guide 130 disposed at a bottom end portion of the vertical bar 110.

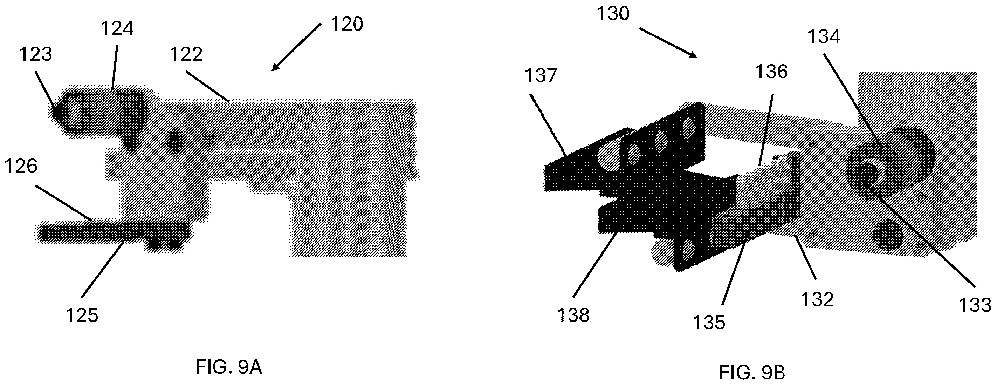

FIG. 9A is a detailed view of the first (upper) strand guide 120. The first strand guide 120 includes a horizontally extending support bar 122, and a support rod 123 and a yarn guide mount 125 attached to the support bar 122. Wheels 124 are mounted on the support rod 123. The yarn guide mount 125 includes screw holes (not shown), and yarn guides 126 are attached to the yarn guide mount 125 with use of the screw holes. The support rod 123 is arranged above the yarn guide mount 125 so that elastic strands leaving the wheels 124 are aligned with the yarn guides 126 and thus the elastic strands are turned vertically downwards.

FIG. 9 B is a detailed view of the second (lower) strand guide 130. The second strand guide 130 includes a horizontally extending support bar 132, and a support rod 133 and a yarn guide mount 135 attached to the support bar 132. Wheels 134 are mounted on the support rod 133. The yarn guide mount 135 includes screw holes (not shown), and yarn guides 136 are attached to the yarn guide mount 135 with use of the screw holes. The yarn guide mount 135 is arranged distally from the support rod 33 so that elastic strands leaving the wheels 134 are aligned horizontally with the yarn guides 136 and thus the elastic strands are turned from vertical to horizontal. The lower strand guide 130 also includes brushes 137, 138 that catch strands that have snapped so that the snapped strands can be easily located and replaced.

A linear actuator has a rail holding sets of yarn guides and flat rollers on which the elastic strands travel to a laminating system. The linear actuator has a bar or rail that slidingly engages stack up configuration plates. At points in the process, elastic strands get close together and may be difficult to re-thread. Additionally, the angle of the stack up can be difficult for an operator to see and re-string. The stack up plates are slidingly disengaged with the linear actuator so that groups of elastic strands can be separated when re-stringing the system.

The mechanism includes two pneumatic devices: one is linear to collect the individual slides, and the second one rotates to return the device to the angle required during production. The rotation for access to the device is manually actuated by the operator. Being that a 5' tall individual has a line of sight lower than a 7' operator, each angle can be achieved as desired, for a friendly ergonomic.

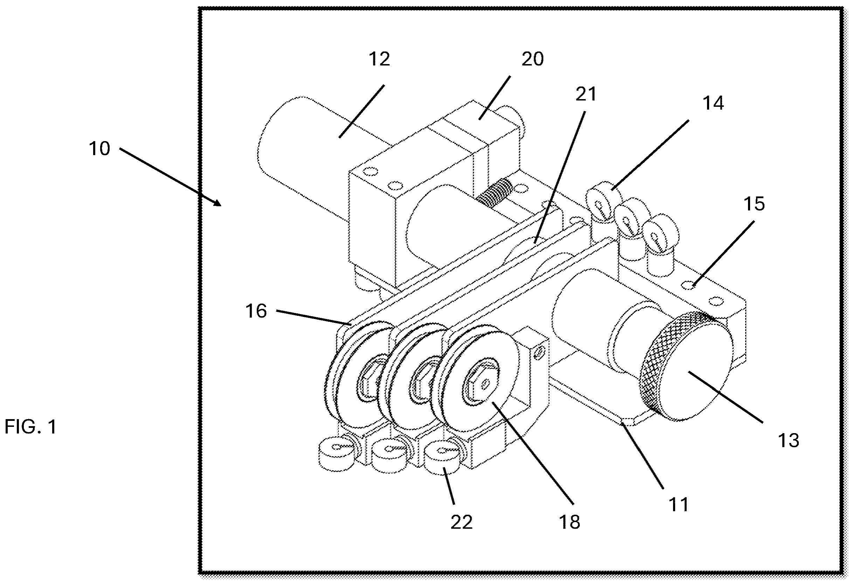

FIG. 1 shows a stack plate, generally designated by reference number 10, according to an exemplary embodiment of the present invention. The stack plate 10 includes a base block 11, a rod 12, yarn guides 14, pitch plates 16, and wheels 18. There is typically one stack plate 10 for each set of elastic strands going into the elastic panel lamination station. The stack plate 10 may hold from 1 to 8 or from 2 to 6 elastic strands. The lengths of the pitch plate 16 sets the pitch of the elastic strands. For example, some strands are 4 mm apart, while others are 8 mm apart. The distance between strands (pitch) can vary based on product need. In exemplary embodiments, elastic strands enter the stack plate at 9 mm apart.

The base block 11 is configured to support other components of the stack plate 10. In this regard, the rod 12 may be held in place on the base block 11 by, for example, bolting the rod 12 to a portion of the yarn guide mount 20. In exemplary embodiments, the rod 12 is threaded to allow for threaded attachment of a removable end portion 13. The removable end portion 13 enables other parts to be attached onto the rod.

In exemplary embodiments, the base block 11 is configured to allow for attachment of the yarn guides 14. For example, the yarn guide mount 20 may include screw holes 15 into which the individual yarn guides 14 may be screwed. Yarn guides 14 have openings that may range from about 1 mm to about 5 mm or about 1.5 mm to about 3 mm or 2 mm in diameter. The diameter of the opening may depend on the decitex (weight in grams per 10,000 meters) of the elastic strand. Suitable yarn guides are available from Yuasa Yarn Guide Engineering Company, Ltd. (Nagoya, Japan).

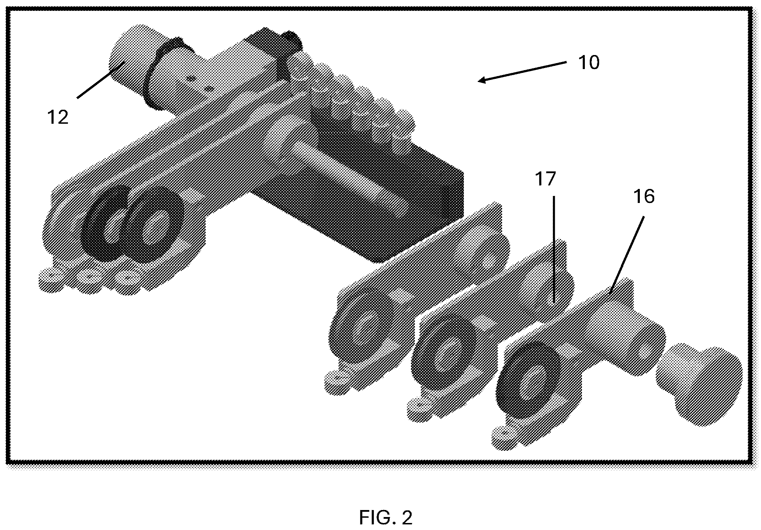

As shown in FIG. 2, to assemble the stack plate 10, the pitch plates 16 are slid onto the rod 12. In this regard, the pitch plates 16 may have openings 17 that allow the pitch plates 16 to be slid onto the rod 12. The length of the pitch plates 16 sets the pitch of the elastic strands.

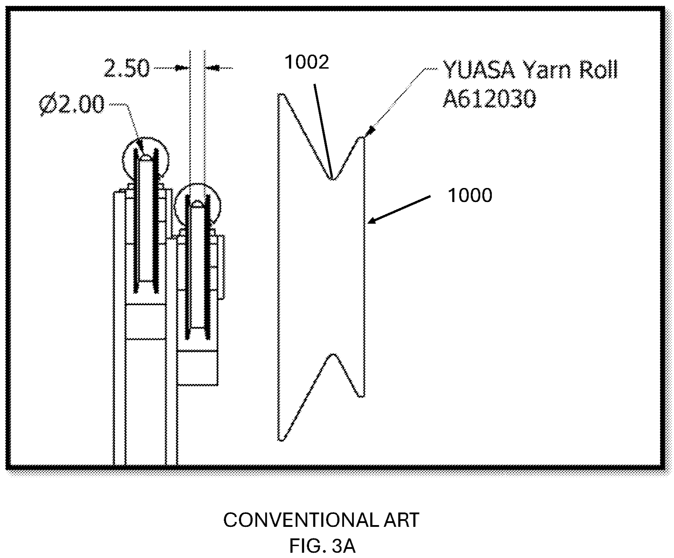

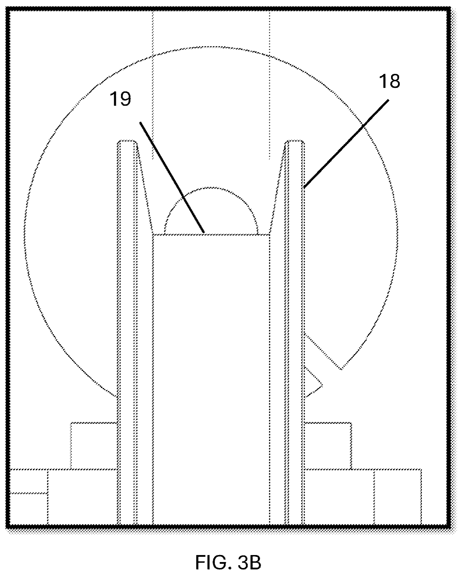

As shown in FIG. 3A, conventional wheels 1000 have a generally v-shaped groove 1002 with a round bottom in which the elastic strand runs. The elastic strands may interact with the sides of the groove 1002, causing the strands to twist and turn and ultimately break or come off of the wheel. Additionally, the shape of the groove can weaken or separate splices of elastic strands. Specifically, it is believed that the continuous twisting of the elastic strands as it travels downstream can cause the weakness and separation of a splice knot. As shown in FIG. 3B, each wheel or roller 18 according to exemplary embodiments of the present invention has a core portion the perimeter of which forms a flat inner surface 19. The flat inner surface 19 prevents the twisting and turning of the strands. The width of the flat inner surface 19 of the wheel or roller may range from about 1 mm to about 5 mm or about 2 mm to about 4 mm or 2.5 mm depending on the decitex of the elastic strands. The width of the flat surface must be wider than the yarn guide inner diameter to prevent strands from contacting the sides of the wheels. Spacers 21 are placed between the pitch plates 16, thereby providing sufficient space for the wheels 18 to be positioned between the pitch plates 16. In exemplary embodiments, the spacers 21 may be portions of the rod 12 or may be extensions of the pitch plates 16. Each wheel or roller 18 may be rotatably attached to a proximal portion of a corresponding one of the pitch plates 16 with the aid of ball bearings. Entry yarn guides 14 are attached to the stack plate 10 just below a corresponding one of the wheels 18. During operation, an elastic strand passes through a corresponding entry yarn guide 14 and over a corresponding wheel 18 that is in line with a corresponding one of the exit yarn guides 22 (in some cases turning 90 degrees), then passes through the corresponding exit yarn guide 22.

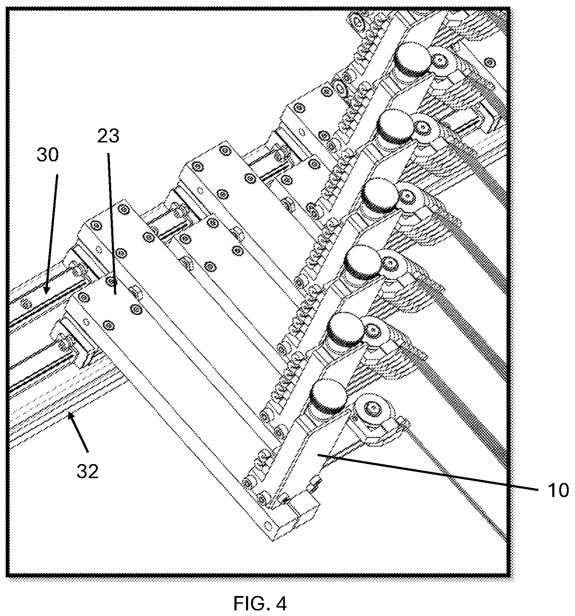

There is typically at least one stack plate for each set of stacked elastic core stands. Core stands are typically aligned perpendicular to the machine direction of the manufacturing machine. As shown in FIG. 4, each stack plate 10 is disposed on a base plate 23, which in turn is attached to a ball bearing carriage slidably mounted on a guide rail 32 of a linear actuator 30. The linear actuator 30 is configured to move the groups of stack plates along the guide rail 32 to direct the elastic strand stands to the lamination station. The base plates 23 may be slidingly disengaged with the guide rail 32 of the linear actuator 32, such that the plates may be separated to facilitate replacing broken strands. The length of each base plate 23 is different based on the number of elastic strands required to make a finished product. A solenoid may be used to control collapsing of the stack plates 10 or freeing for manual sliding motion of the base plates 23. When running, the solenoid locks the base plates 23 such that they may not be moved manually and the linear actuator 30 can operate to bring the base plates 23 together in a collapsing motion. When a strand breaks and the system is down, the solenoid activates the linear actuator to open to enable manual movement of the base plates 23 such that the base plate or plates 23 may slide to create space for the machine operator to restring the system.

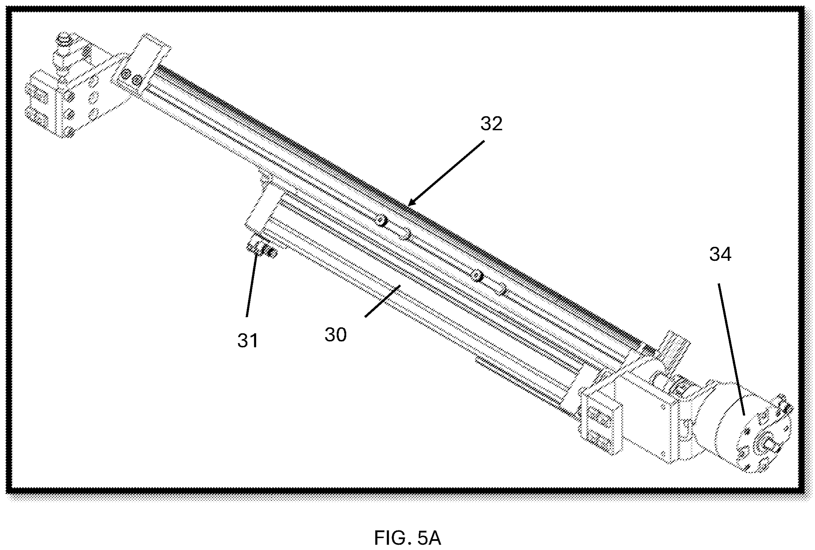

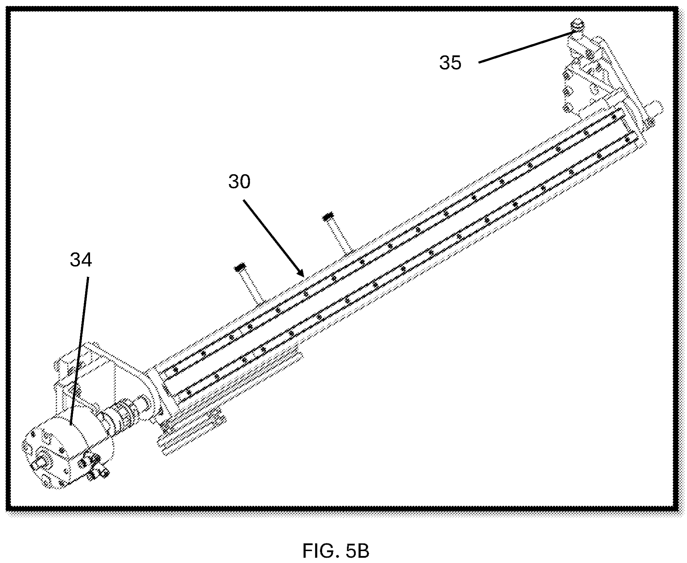

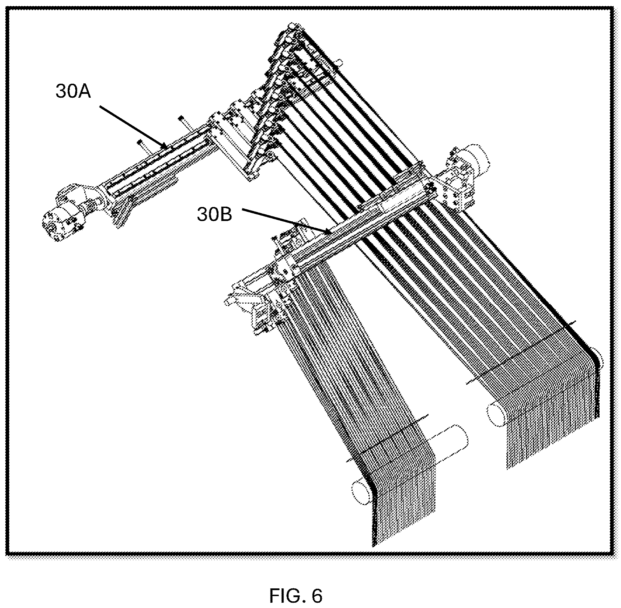

Where the elastic strands reach the lamination station, the stack plates 10 for each set of srtands are attached to the linear actuator 30 and guide rail 32. At this point, the strands are turned 90 degrees so that they enter into the lamination area parallel with the nonwoven materials between which they are laminated. The elastic strands are typically retained between the nonwoven materials with adhesive, ultrasonic bonding, thermal bonding and the like. An important aspect of collapsing the base plates 23 to contact one another is to achieve perfectly parallel strands, while forming the required pitch of the laminated web. As shown in FIGS 5 A and 5 B, linear actuator 30 has an air flow control valve 31 and the linear actuator 30 may include an air cylinder for pneumatically and automatically closing the device upon startup. Alternatively, stepper motors, servo motors and the like may be used to enable similar functions. A rotary cylinder 34, or other rotary actuating element, enables the rail 32 to be rotated up to 60 degrees or up to 40 degrees. In this case, the rotary motion activates to return the unit to the correct production angle. In exemplary embodiments, an operator can manually rotate the unit to enable better visibility of the stack plates 10 and facilitate replacing broken elastic strands. A shock absorber 35 is positioned on an upper surface to control the end of stroke when the actuator 30 is closed. There may be more than one linear actuator 30, for example one linear actuator for elastic strands being fed to make a front elastic panel and another linear actuator for elastic strands being fed to make a back elastic panel. Linear actuators are available for example from Festo US Corporation (Islandia, New York, USA). FIG. 6 shows one linear actuator 30A with elastic strands feeding a lamination station to make back panels and a second linear actuator 30B with elastic strands feeding the lamination station to make front panels.

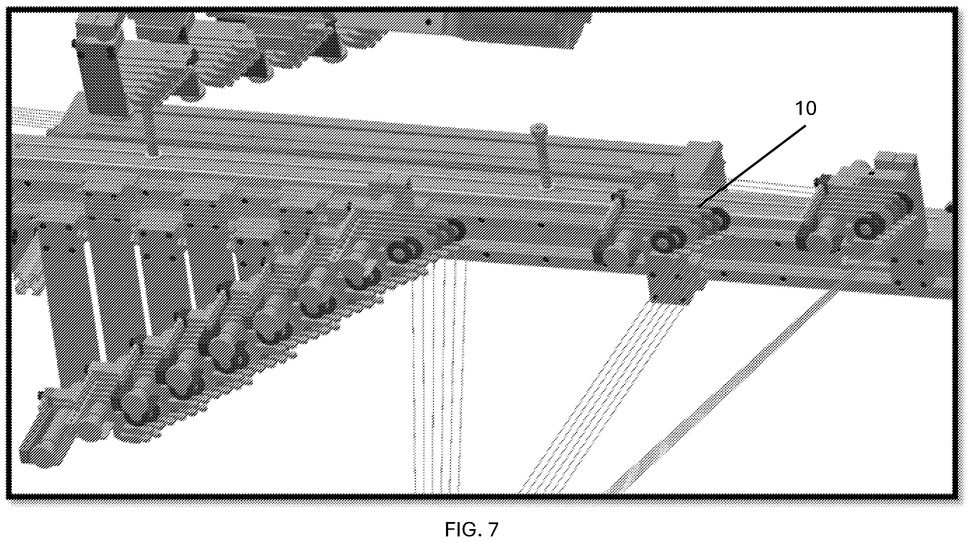

FIG. 7 shows a stack plate 10 that was moved away from the remaining stack plates to facilitate restringing an elastic strand. Each stack of core stands may be color coded and the alignment and lowering system for each of the core stands may have components that are color coded the same. For example, the alignment and lowering system for a first set of stands may have purple parts and the stack plate 10 in the line for the same set of strands may also be purple, the alignment and lowering system parts and stack plates 10 for a second stack of stands may be blue, and so on. In general, elastic strands exit the stand and move vertically to a common height at which point they engage the alignment and lowering system and are turned 90 degrees to run horizontally. The common height is not critical, but is set to facilitate easily reaching and replacing broken elastic strands. The lowered height also enhances safety and improves parallel alignment of the strands.

As the lamination station may make panels side by side, one set of elastic strands may run along a right side of elastic core stand stacks and a separate set of elastic strands may run along a left side of elastic core stand stacks (both on an operator side of the manufacturing machine). The two sides of stacks are typically distanced such that an operator can easily walk between the stacks, access the lamination station and restring the strands. Alternatively, one set of elastic strands for one panel may be fed from the operator side of the machine and the other set for the other panel may be fed from the drive side of the machine or both sets of strands may be fed from the drive side of the machine. Front and back panel feeding configurations may vary, such as, for example, feeding from both sides.

### Example

A system for feeding elastic strands was made using 6 stacks of elastic core stands, each having 6 stacked stands for a total of 36 elastic strands. The elastic strands were 800 decitex from LYCRA Company for portions of the panel and 540 decitex for other portions of the panel. The first stand was fed to a light blue alignment and lowering system with 6 wheels or rollers, each having a flat inner surface of 2.5 mm, one elastic strand in each wheel. Yuasa yarn guides (2 mm inner diameter) were used at the entrance to and exit from the stack plate. The strands were fed through the entrance yarn guide, over the wheel or roller and through the exit yarn guide. At the linear actuator, the length of the pitch configuration plates was selected to provide 4 mm spacing between elastic strands. The height from the ground that the elastic strands were run was 5 feet.

At the second stack of elastic core stands, the same configuration was provided, but a second alignment and lowering system (this one colored purple) was used for this set of elastic strands. Subsequent stacks of core stands had gold, orange, green and then red stack plates. The stack plates were attached such that each set of elastic strands extended further from the source and traveled parallel towards the lamination station. At the lamination station, a rail associated with a Festo linear actuator had six stack plates with the same color coding above (one for each set of 6 strings) attached next to each other. The elastic strands were fed into the entrance yarn guides, over the flat wheels or rollers, out through the exit yarn guides and to the nonwovens for forming elastic panels. Entering the stack plates, the elastic strands were perpendicular to the machine and exiting the stack plates, the elastic strands were turned to be parallel with the machine. When compared to conventional elastic strand feeding, this process and equipment resulted in far fewer elastic strands breaking and significantly faster restringing when a strand broke. For example, with conventional elastic strand feeding, the time to restring may be 20 to 30 minutes. With the equipment of the present invention. restringing may take 5 to 10 minutes. The ability to open the linear actuator significantly helps reduce the time to restring. The height the elastic strands were traveling at also helps reduce time and safety as the operator does not need to climb a ladder to access the strands.

Now that embodiments of the present invention have been shown and described in detail, various modifications and improvements thereon can become readily apparent to those skilled in the art. Accordingly, the exemplary embodiments of the present invention, as set forth above, are intended to be illustrative, not limiting. The spirit and scope of the present invention is to be construed broadly.

## Claims

1. A stack plate for directing elastic strands to a lamination station of an absorbent article manufacturing line, comprising:
a base block;
two or more entry yarn guides disposed on the base block and each configured to receive a corresponding elastic strand of two or more elastic strands;
two or more wheels disposed on the base block and each corresponding to one of the two or more entry yarn guides and over which the corresponding elastic strand is guided as the two or more wheels rotate;
two or more exit yarn guides disposed on the base block and each corresponding to one of the two or more entry yarn guides and to one of the two or more wheels, each of the two or more exit yarn guides configured to guide the corresponding elastic strand away from the stack plate; and
two or more pitch plates disposed on the base block and each corresponding to one of the two more entry yarn guides, one of the two or more wheels and one of the two or more exit yarn guides, each of the two or more pitch plates extending between the corresponding one of the two or more wheels and the corresponding one of the two or more exit yarn guides, each of the two or more pitch plates having lengths that differ from one another so as to determine a distance between the two or more elastic strands.

2. The stack plate of claim 1, further comprising a rod extending perpendicular to the two or more pitch plates, the rod configured to hold the two or more pitch plates in position on the base block.

3. The stack plate of claim 2, wherein each of the two or more stack plates comprise openings, and the rod extends through the openings.

4. The stack plate of claim 2, wherein the rod comprises a removeable end portion to allow for placement of the two or more pitch plates on the rod.

5. The stack plate of claim 1, wherein each of the two or more wheels are disposed on the corresponding one of the two or more pitch plates.

6. The stack plate of claim 1, wherein the two or more entry yarn guides, the two more wheels and the two or more exit yarn guides are positioned so that the two or more elastic strands are turned 90 degrees between the two or more entry yarn guides and the two or more exit yarn guides.

7. The stack plate of claim 1, wherein the two or more wheels comprise an inner core member that forms a flat surface on which the two or more elastic stands are conveyed as the two or more wheels rotate.

8. The stack plate of claim 1, wherein the stack plate is configured for placement on a linear actuator.

9. An elastic strand conveyance system comprising:
(A) one or more alignment and lowering systems for directing elastic strands to one or more stack plates of a lamination station of an absorbent article manufacturing line, , each of the one or more stack plates comprising:
(i) a base block;
(ii) two or more entry yarn guides disposed on the base block and each configured to receive a corresponding elastic strand of two or more elastic strands;
(iii) two or more wheels disposed on the base block and each corresponding to one of the two or more entry yarn guides and over which the corresponding elastic strand is guided as the two or more wheels rotate;
(iv) two or more exit yarn guides disposed on the base block and each corresponding to one of the two or more entry yarn guides and to one of the two or more wheels, each of the two or more exit yarn guides configured to guide the corresponding elastic strand away from the stack plate; and
(v) two or more pitch plates disposed on the base block and each corresponding to one of the two more entry yarn guides, one of the two or more wheels and one of the two or more exit yarn guides, each of the two or more pitch plates extending between the corresponding one of the two or more wheels and the corresponding one of the two or more exit yarn guides, each of the two or more pitch plates having lengths that differ from one another so as to determine a distance between the two or more elastic strands;
(B) one or more linear actuators to which the one or more stack plates are operatively attached to allow for linear movement of the one or more stack plates.

10. The elastic strand conveyance system of claim 9, wherein each of the one or more linear actuators comprises a rail on which a corresponding one of the one or more stack plates is conveyed in a linear motion by the linear actuator.

11. The elastic strand conveyance system of claim 10, wherein the one or more stack plate are linearly disengageable from the rails of the one or more linear actuators to allow for manual movement of the one or more stack plates.

12. The elastic strand conveyance system of claim 9, wherein the one or more stack plates comprise a plurality of stack plates.
